# FASCICULE DE BREVET EUROPEEN

(11) **EP 3 393 393 B1**
(45) Date de publication et mention de la délivrance du brevet: **02.06.2021**
(21) Numéro de dépôt: 16708178.5
(22) Date de dépôt: 07.03.2016
(51) Int. Cl.: A61C 1/14

(54) **INSTRUMENT DENTAIRE ET/OU CHIRURGICAL AVEC UNE CARTOUCHE ET UNE CARTOUCHE CORRESPONDANTE**
ZAHNMEDIZINISCHES UND/ODER CHIRURGISCHES INSTRUMENT MIT EINER KARTUSCHE UND ENTSPRECHENDE PATRONE
DENTAL AND/OR SURGICAL INSTRUMENT WITH A CARTRIDGE, AND A CORRESPONDING CARTRIDGE

(30) Priorité: 07.03.2015 CH 3152015
(43) Date de publication de la demande: 31.10.2018
(73) Titulaire: Bien-Air Holding SA, 2504 Bienne (CH)
(72) Inventeur: JUILLERAT, Sébastien, 2740 Moutier (CH); SCHENK, Marc, 2525 Le Landeron (CH)
(74) Mandataire: BOVARD AG
(86) Numéro de dépôt international: PCT/EP2016/054796
(87) Numéro de publication internationale: WO 2016/142342

(56) Documents cités:
- EP-A1- 0 527 473
- EP-A1- 1 378 207
- EP-A2- 0 724 867
- JP-A- H0 838 509
- US-A1- 2011 183 286

## Description

### Domaine technique de l'invention

La présente invention se rapporte au domaine des instruments médicaux, plus particulièrement des instruments dentaires et/ou chirurgicaux. De manière plus spécifique, la présente demande se rapporte à une amélioration au niveau de la conception de l'instrument dentaire et/ou chirurgical.

Dans le cadre de cette demande, l'expression « instrument » est utilisée comme un terme générique désignant tout outil de travail d'un praticien, notamment d'un dentiste ou d'un chirurgien. De même, l'expression « moteur » désigne de façon universelle tout dispositif apte à créer un mouvement mécanique (notamment un mouvement rotatif, linéaire, oscillatoire, ultrasons, etc.), l'expression « pièce à main » désigne globalement le dispositif qui transmet ou qui transforme un tel mouvement mécanique, et l'expression « tête » désigne la partie de la pièce à main constituée des éléments de transmission du mouvement selon un angle prédéfini, des éléments de guidage, et des éléments de serrage de l'outil.

### Etat de la technique

On connaît déjà les instruments dentaires et/ou chirurgicaux comprenant les pièces à main de type « turbine » (avec un entraînement par air) ou de type « contre-angle » (avec un entraînement par moteur et par engrenage). Dans les deux cas, les pièces à main comprennent essentiellement un système de serrage (et d'entrainement) de l'outil qui est généralement monté sur des paliers à billes.

Comme les systèmes de serrage de l'outil tournent à très haute vitesse, la précision mécanique du positionnement de ces systèmes de serrage de l'outil est d'une importance essentielle pour la sécurité, la qualité, les performances de l'instrument et le confort du praticien et celui du patient.

La conception des systèmes actuels du marché est caractérisée en ce que les diamètres de guidage des paliers à billes ne sont pas constitués du même alésage (cylindre), et/ou par une complexité accrue de montage pour la précharge des roulements. Par conséquent, les systèmes actuels présentent les défauts suivants :
- Les erreurs géométriques (notamment dans l'alignement et dans la concentricité) participent à dégrader l'excentricité dynamique de l'outil et à augmenter les phénomènes vibratoires ;
- Les paliers à billes sont généralement préchargés à l'aide d'un élément ressort, afin de supprimer le jeu axial de l'outil. Par conséquent, une erreur de désalignement va engendrer une répartition inhomogène de la force de précharge des paliers et accélérer leur dégradation ;
- Pour les pièces à main de type « contre-angle », la qualité d'engrènement entre les pignons menants et menés est primordiale pour garantir un niveau vibro-accoustique de qualité et garantir un meilleur rendement pour la transmission des efforts ;
- Les systèmes dont les alésages de guidage des paliers à billes sont directement usinés dans la tête de la pièce à main sont extrêmement sensibles à la résistance aux chocs. Un choc sur le côté de la tête de la pièce à main peut notamment déformer localement la matière de la tête et supprimer le jeu radial du palier. Par conséquent, cela peut engendrer la détérioration complète du palier ;
- Les impacts opérationnels se situent au niveau du nombre de pièces très précises à usiner puis au niveau de la complexité de montage ; et
- Lorsque le service après-vente est organisé de manière à fonctionner sur plusieurs sites (ce qui est assez souvent le cas), le changement des pièces d'usures (p.ex. des paliers à billes) demande des outillages spéciaux et une formation des techniciens pour chaque site.

Un exemple d'un document illustrant les solutions connues à ce jour et présentant les défauts susmentionnés est EP 0 527 473 où une erreur géométrique importante peut se produire au niveau de l'alignement et de la concentricité des paliers à billes. Dans le document EP 1 378 207, les deux paliers à billes sont guidés sur deux alésages différents, créant ainsi un risque de mauvaise concentricité, et ayant un mécanisme de précharge des paliers à billes assez complexe au niveau du nombre de composants, de la cinématique de montage, de la qualification du personnel et de l'outillage spéciaux. Finalement, DE 43 24 493 présente aussi un mécanisme de précharge des paliers à billes assez complexe au niveau du nombre de composants, de la cinématique de montage, de la qualification du personnel et de l'outillage spéciaux, puis aussi avec une grande susceptibilité aux chocs.

### Exposé sommaire de l'invention

Il est donc un but de la présente invention de proposer un nouvel instrument dentaire et/ou chirurgical qui ne présente pas ces défauts des instruments connus à ce jour. De manière plus spécifique, un but de la présente invention est de proposer un instrument dentaire et/ou chirurgical permettant de diminuer au maximum les erreurs géométriques, d'améliorer la répartition de la force de précharge des paliers et la qualité d'engrènement des paliers, d'apporter une résistance au choc supérieure, de diminuer le nombre de pièces précises à usiner puis monter, et finalement d'améliorer la flexibilité pour le service après-vente.

A cette fin, la présente invention consiste essentiellement à prémonter les éléments de la tête dans une « cartouche » (ou dans une « capsule »), la géométrie de la cartouche étant conçue de manière à optimiser le positionnement des axes de transmission du mouvement et d'alignement des éléments de guidage et de serrage de l'outil. En même temps, une cartouche selon la présente invention peut également être facilement remplacée, sans recourir à un outillage ou à des procédés spéciaux.

Plus particulièrement, la présente invention est décrite dans les revendications indépendantes, les revendications dépendantes et la description mettant à disposition les modes de réalisation préférés.

Selon un premier aspect de la présente invention, elle est relative à une cartouche amovible qui peut être insérée dans la tête d'un instrument dentaire et/ou chirurgical selon les caractéristiques de la revendication 1, comprenant une douille avec un alésage intérieur cylindrique unique et une ouverture latérale, et un sous-ensemble avec un rotor, un premier palier à billes, un deuxième palier à billes, ainsi qu'un porte-outil. L'ouverture latérale de la douille est adaptée pour la connexion entre un arbre menant d'un instrument dentaire et/ou chirurgical et le rotor entraînant en rotation le porte-outil, et le rotor comprend une denture extérieure pouvant engrener avec une denture correspondante de l'arbre menant.

Selon cette configuration, il n'y a qu'un seul alésage pour monter et guider les deux paliers à billes supportant la partie rotative, ce qui permet de minimiser toute erreur de positionnement des paliers et tout problème lié à ces erreurs, comme sera montré par la suite.

Comme elle contient tous les éléments « actifs » de la tête de l'instrument, une telle cartouche permet un remplacement facile.

Selon un deuxième aspect, la présente invention concerne un instrument dentaire et/ou chirurgical comprenant un moteur entraînant un outil par le biais d'un porte-outil situé dans la tête de l'instrument dentaire et/ou chirurgical, ainsi qu'une telle cartouche amovible, de telle sorte que le porte-outil est supporté dans la tête de l'instrument dentaire et/ou chirurgical par le premier palier à billes et le deuxième palier à billes de la cartouche amovible.

Une tel instrument dentaire complet pourvu d'une telle cartouche amovible conférant des propriétés de guidage améliorées reflète par ailleurs la configuration modulaire conférée par la présente invention.

### Brève description des dessins

La présente invention sera mieux comprise à la lecture de la description qui va suivre, donnée à titre d'exemple et faite en référence au dessin dans lequel :
- figure 1 est une vue schématique en coupe d'un instrument dentaire et/ou chirurgical selon un mode de réalisation de la présente invention ;
- figure 2 avec les sous-figures 2A à 2E est une vue schématique avec une séquence de montage d'une cartouche dans le sens de la présente invention ; et
- figure 3 est une vue en coupe de la tête d'un instrument dentaire et/ou chirurgical selon un mode de réalisation de la présente invention.

### Description détaillée de l'invention

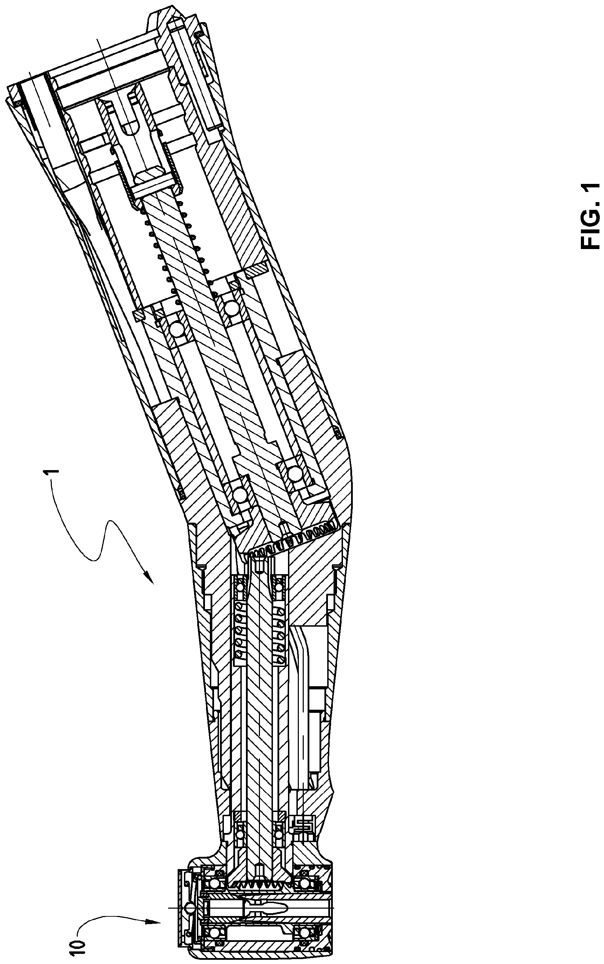

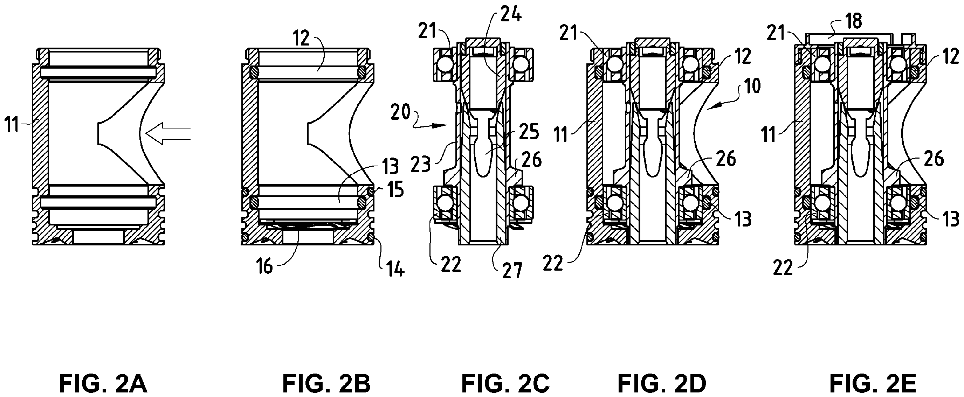

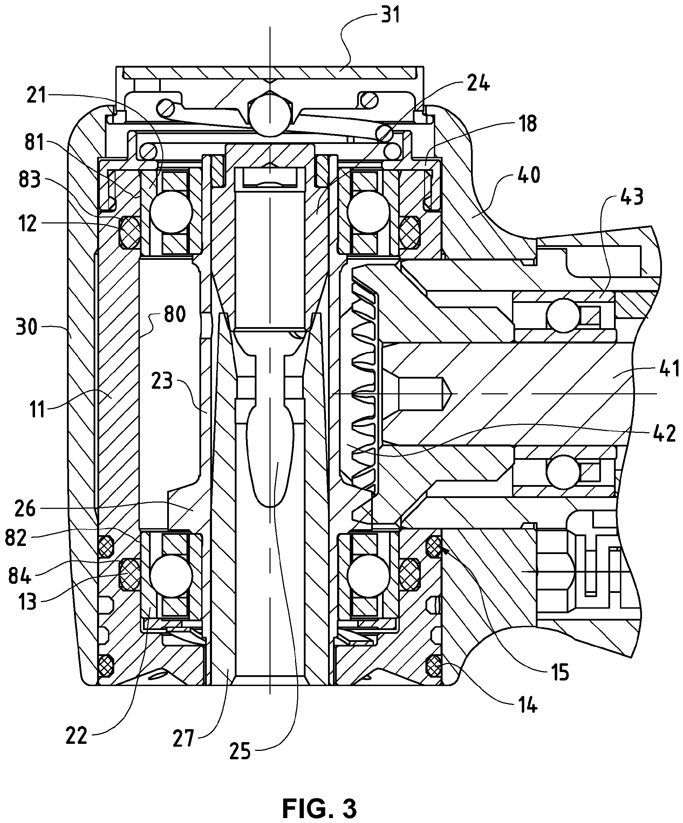

Un instrument dentaire et/ou chirurgical 1 selon un mode de réalisation de la présente invention est représenté de manière schématique à la figure 1 et un détail de sa structure aux figures 2 et 3. Dans cet exemple, il s'agit notamment d'un instrument dentaire et/ou chirurgical rotatif en « contre-angle », donc d'un instrument utilisé pour porter et entraîner une fraise dentaire qui est ensuite utilisée pour évincer les tissus des dents ramollis par la carie. Cet instrument dentaire 1 comprend notamment une cartouche 10 selon un mode de réalisation de la présente invention qui sera décrite plus en détail plus loin. Bien entendu, la présente invention se rapporte également à des instruments chirurgicaux présentant la même structure.

Un but important de la présente invention est la mise à disposition d'un instrument dentaire et/ou chirurgical dans lequel l'erreur géométrique est fortement diminuée par rapport aux solutions existantes. En effet, pour un fonctionnement et une durée de vie optimale des paliers qui portent le système de serrage et de guidage de l'outil (notamment la fraise), ces derniers doivent

être montés de manière à ce que l'erreur de désalignement / concentricité relative en les deux soit la plus petite possible.

A cette fin, la présente invention propose de monter et guider les deux paliers dans un même alésage (c'est-à-dire dans un cylindre), ce qui résulte en une minimisation de l'erreur géométrique et autres avantages cités ci-dessous. Dans le cas contraire, chaque portée ou pièce supplémentaire peut doubler cette erreur relative.

Pour arriver à une telle solution, la présente invention propose à créer ce cylindre de montage et de guidage des paliers à billes dans une douille appelée diffuseur et de monter les éléments de transmission de mouvement, ainsi que les éléments de guidage et de serrage de l'outil dans cette douille. Ensuite, la douille avec les éléments montés est fermée et sécurisée avant qu'elle ne soit insérée dans la tête de l'instrument dentaire et/ou chirurgical.

Figure 2 avec les sous-figures 2A à 2E montre la séquence de montage de la cartouche selon un mode de réalisation de la présente invention.

Une douille 11 est d'abord mise à disposition comme illustré à la figure 2A. Cette douille 11 contient un alésage cylindrique 80 à l'intérieur, ainsi qu'une ouverture latérale (illustrée par une flèche) qui sert à connecter le rotor 23 de la cartouche 10 à l'arbre menant 41 de l'instrument dentaire et/ou chirurgical 1 (illustré à la figure 3). Cet alésage cylindrique 80 sert à monter et guider à la fois le premier palier à billes et le deuxième palier à billes qui seront décrit plus tard.

Dans cette douille 11, l'alésage 80 avec les portées 81, 82 de guidage des paliers ainsi que les saignées 83, 84 qui logent les joints d'amortissement 12, 13 (cf. la figure 2B) est usiné lors d'une même opération. Par conséquent, l'erreur de concentricité entre les deux paliers après leur montage est minimisée et la compression des joints d'amortissement 12, 13 sur les paliers est homogène. A la prochaine étape, illustrée à la figure 2B, les joints d'amortissement 12, 13 sont montés dans les saignées 83, 84 prévues à cette fin. Aussi, un ressort de précharge des paliers 16 est également monté dans la douille 11.

Le sous-ensemble 20 avec un rotor 23 est monté en parallèle, tel que visible à la figure 2C. Ce sous-ensemble 20 comprend les deux paliers à billes 21, 22, ainsi que les éléments de serrage et de guidage de l'outil (ou un porte-outil) 24, 25, 27. Une denture extérieure (ou pignon) 26 est prévue sur le rotor 23. Cette denture extérieure 26 engrène avec la denture correspondante 42 de l'arbre menant 41 afin d'entraîner ce sous-ensemble 20, et donc également l'outil lorsque l'instrument dentaire et/ou chirurgical 1 est en fonction, en rotation (cf. figure 3). Cette denture 26 peut former partie intégrante du rotor 23, mais peut aussi être réalisée comme pièce séparée et chassée sur le rotor 23.

Le sous-ensemble 20 assemblé, il est monté dans la douille 11 avec les joints d'amortissement 12, 13 prémontés pour créer la cartouche 10. A la fin de ce montage, la cartouche 10 est verrouillée à l'aide d'un écrou 18.

Figure 3 montre la tête de l'instrument dentaire et/ou chirurgical 1 dans l'état monté. Il est visible que la cartouche 10 est insérée dans l'espace libre crée par la paroi 30 de la tête, puis bloquée dans la tête par un mécanisme de blocage standard 31. Il est aussi visible à la figure 3 que la denture (le pignon) 42 de l'arbre menant 41 (qui est entrainé par un moteur qui n'est pas représenté) engrène avec la denture 26 dans la cartouche 10 afin d'entraîner les éléments de serrage et de guidage de l'outil 24, 25, 27 supportés par les paliers à billes 21, 22 en rotation. Cet arbre menant 41 est également supporté par les paliers à billes dont un seul (no. de référence 43) est visible à la figure 3.

En addition à cette transmission de l'énergie d'entraînement, l'extrémité de l'arbre menant 41 sert également de positionnement et de verrouillage de la cartouche 10. En effet, et comme visible à la figure 3, l'extrémité de l'arbre menant 41 vient s'insérer dans l'ouverture latérale (illustrée par une flèche à la figure 2A) et peut ainsi bloquer la cartouche 10 dans la position correcte.

En addition à l'amélioration de la géométrie et la diminution de l'erreur correspondante, l'instrument dentaire et/ou chirurgical 1 selon la présente invention présente aussi une amélioration au niveau de la précharge des paliers. En effet, ce point est intimement lié à l'erreur géométrique citée ci-dessus. Si les deux paliers à billes 21, 22 ne sont pas concentriques, la force appliquée pour la précharge ne va pas être distribuée uniformément sur les composants de chaque palier 21, 22. Plutôt, cette force va se diviser en composantes qui peuvent engendrer des contraintes parasites accélérant la dégradation de chaque palier 21, 22 en dynamique.

De même, et grâce au verrouillage de la cartouche 10 en place par l'extrémité de l'arbre menant 41, la qualité d'engrènement est également améliorée dans un instrument dentaire et/ou chirurgical 1 selon la présente invention. En effet, la perpendicularité des deux axes de transmission 41, 23 est également liée à la précision obtenue à l'usinage de la douille (ou du diffuseur) 11. La précision de cette pièce va par conséquent directement influencer le positionnement relatif entre les deux axes de transmission 41, 23 et ainsi améliorer la qualité d'engrènement. Bien entendu, il serait également imaginable de prévoir un instrument dentaire et/ou chirurgical 1 dans lequel les axes de transmission 41, 23 ne sont pas perpendiculaires, mais inclinés l'un par rapport à l'autre d'un angle prédéterminé, notamment avec le but d'améliorer l'ergonomie de l'instrument 1 et/ou pour répondre aux besoins anatomiques.

Un autre avantage de l'instrument dentaire et/ou chirurgical 1 selon la présente invention est l'amélioration de la résistance aux chocs. En cas de chute, la zone de contact de l'instrument 1 est très souvent son extrémité distale. Dans l'instrument dentaire et/ou chirurgical 1 selon la présente invention, l'énergie du choc va d'abord être absorbée par le matériau de la tête (c'est-à-dire par l'enveloppe extérieure) 30, puis par le matériau de la cartouche 10 elle-même (donc la paroi 11). La solution retenue permet donc d'obtenir un effet multicouche grâce aux diverses rigidités des matériaux et ainsi diminuer la propagation des déformations pouvant détruire les paliers à billes 21, 22.

Aussi, la présente invention propose une rationalisation au niveau du nombre de pièces qui composant l'instrument dentaire et/ou chirurgical 1. De plus, les principaux avantages sont les suivants :
- La qualité de guidage des paliers 21, 22 et de perpendicularité des axes de transmission 41, 23 réside dans la précision d'usinage de la douille (du diffuseur) 11. Par conséquent, la qualité globale du mécanisme repose sur un composant pouvant être usiné sur des équipements d'usinage standards ;
- La douille (le diffuseur) 11 peut donner lieu à des optimisations au niveau de la conception, des matériaux et de la mise en œuvre de ce composant ;
- Le verrouillage de la cartouche 10 grâce à un écrou 21 permet d'assembler au montage des sous-ensembles pouvant être stockés. La précharge n'est ainsi réglée qu'une seule fois ;
- Lors du montage, la cartouche 10 s'oriente (axialement et angulairement) très facilement grâce à l'alésage de précision du diffuseur 11 et le diamètre extérieur du sous-ensemble de l'arbre menant 23. La qualité d'engrènement est donc garantie immédiatement, sans passer par une opération complémentaire de réglage (ou revisitage) du positionnement relatif entre le pignon menant 42 et le pignon mené 26.
- Finalement, le montage de la cartouche 10 reste une opération à forte valeur ajouté, au niveau du réglage de la fonction de précharge. Cependant, l'avantage de pouvoir verrouiller la cartouche 10 dans son état assemblé permet de pouvoir stocker ces dernières puis de les livrer en tant que sous-ensemble pour le service après-vente (SAV). Le technicien SAV peut alors tout simplement retirer la cartouche 10 défectueuse puis la remplacer très rapidement, à l'aide d'un minimum d'outillage.

Dans ce qui précède, l'invention a été décrite d'abord en termes généraux et ensuite sous forme d'une explication de réalisations pratiques. Bien entendu, l'invention n'est pas limitée à la description de ces modes de mise en œuvre ; il va de soi que de nombreuses variations et modifications peuvent être apportées sans que l'étendue de l'invention qui est définie par le contenu des revendications, ne soit quittée.

## Revendications

1. Cartouche (10) amovible apte à être insérée dans la tête d'un instrument dentaire et/ou chirurgical (1), comprenant
une douille (11) avec un alésage (80) intérieur cylindrique unique et une ouverture latérale, et
un sous-ensemble (20) avec un rotor (23), un premier palier à billes (21), un deuxième palier à billes (22), ainsi qu'un porte-outil (24, 25, 27), l'ouverture latérale de la douille (11) étant adaptée pour la connexion entre un arbre menant (41) d'un instrument dentaire et/ou chirurgical (1) et le rotor (23) entraînant en rotation le porte-outil (24, 25, 27), le rotor (23) comprenant une denture extérieure (26) pouvant engrener avec une denture correspondante de l'arbre menant (41).

2. Cartouche (10) amovible selon la revendication 1, **caractérisée en ce que** l'alésage (80) comprend au moins une première saignée (83) et une deuxième saignée (84), la première saignée (83) servant à loger un premier joint d'amortissement (12) et la deuxième saignée (84) servant à loger un deuxième joint d'amortissement (13).

3. Instrument dentaire et/ou chirurgical (1) comprenant un moteur entrainant un outil par le biais d'un porte-outil (24, 25, 27) situé dans la tête de l'instrument dentaire et/ou chirurgical (1), et une cartouche (10) amovible selon la revendication 1, de telle sorte que le porte-outil (24, 25, 27) est supporté dans la tête de l'instrument dentaire et/ou chirurgical (1) par le premier palier à billes (21) et le deuxième palier à billes (22) de la cartouche (10) amovible.

4. Instrument dentaire et/ou chirurgical (1) selon la revendication 3, **caractérisé en ce que** la cartouche (10) est verrouillée par un écrou (18).

5. Instrument dentaire et/ou chirurgical (1) selon la revendication 3 ou 4, **caractérisé en ce que** la cartouche (10) est positionnée et verrouillée dans la tête de l'instrument dentaire et/ou chirurgical (1) grâce à l'extrémité de l'arbre menant (41).

6. Instrument dentaire et/ou chirurgical (1) selon l'une des revendications 3 à 5, **caractérisé en ce que** l'axe central de l'arbre menant (41) est perpendiculaire à l'axe du rotor (23).

7. Instrument dentaire et/ou chirurgical (1) selon l'une des revendications 3 à 5, **caractérisé en ce que** l'enveloppe extérieure (30) de l'instrument dentaire et/ou chirurgical (1) est réalisée en un matériau différent du matériau de la cartouche (10).

## Patentansprüche

1. Lösbares Modul (10), eingerichtet um in den Kopf eines dentalen und/oder chirurgischen Instruments (1) eingeführt zu werden, umfassend:
eine Hülse (11) mit einer einzigen zylindrischen inneren Bohrung (80) und einer seitlichen Öffnung, und
eine Untereinheit (20) mit einem Rotor (23), einem ersten Kugellager (21), einem zweiten Kugellager (22) und einem Werkzeughalter (24, 25, 27), wobei die seitliche Öffnung der Hülse (11) eingerichtet ist für die Verbindung zwischen einer Antriebswelle (41) und einem dentalen und/oder chirurgischen Instrument (1) und der Rotor (23) den Werkzeughalter (24, 25, 27) in Drehung versetzt, wobei der Rotor (23) eine Aussenverzahnung (26) aufweist, welche mit einer zur Verzahnung der Antriebswelle (41) korrespondierenden Aussenverzahnung in Eingriff gebracht werden kann.

2. Lösbares Modul (10) nach Anspruch 1, **dadurch gekennzeichnet, dass** die Bohrung (80) zumindest eine erste Nut (83) und eine zweite Nut (84) aufweist, wobei die erste Nut (83) vorgesehen ist, um eine erste Dämpfungsdichtung (12) aufzunehmen und die zweite Nut (84) vorgesehen ist, um eine zweite Dämpfungsdichtung (13) aufzunehmen.

3. Dentales und/oder chirurgisches Instrument (1), welches einen Motor zum Antreiben eines Werkzeughalters (24, 25, 27) umfasst, angeordnet in dem Kopf des dentalen und/oder chirurgischen Instruments (1) und ein lösbares Modul (10) nach Anspruch 1, so dass der Werkzeughalter (24, 25, 27) in dem Kopf des dentalen und/oder chirurgischen Instruments (1) von dem ersten Kugellager (21) und dem zweiten Kugellager (22) des lösbaren Moduls (10) gehalten ist.

4. Dentales und/oder chirurgisches Instrument (1) nach Anspruch 3, **dadurch gekennzeichnet, dass** das Modul (10) von einer Mutter (18) arretiert ist.

5. Dentales und/oder chirurgisches Instrument (1) nach einem der Ansprüche 3 oder 4, **dadurch gekennzeichnet, dass** das Modul (10) in dem Kopf des dentalen und/oder chirurgischen Instruments (1) mittels dem Ende der Antriebswelle (41) positioniert und arretiert ist.

6. Dentales und/oder chirurgisches Instrument (1) nach einem der Ansprüche 3 bis 5, **dadurch gekennzeichnet, dass** die zentrale Achse der Antriebswelle (41) senkrecht zu der Welle des Rotors (23) ist.

7. Dentales und/oder chirurgisches Instrument (1) nach einem der Ansprüche 3 bis 5, **dadurch gekennzeichnet, dass** das Aussengehäuse (30) des dentalen und/oder chirurgischen Instruments (1) aus einem anderen Material gefertigt ist als das Material des Moduls (10).

## Claims

1. Removable cartridge (10) suitable for insertion into the head of a dental and/or surgical instrument (1), comprising:
a bush (11) with a single cylindrical inner bore (80) and a lateral opening, and
a sub-assembly (20) with a rotor (23), a first ball bearing (21), a second ball bearing (22) and a tool holder (24, 25, 27), the lateral opening of the sleeve (11) being adapted for the connection between a driving shaft (41) of a dental and/or surgical instrument (1) and the rotor (23) driving the tool holder (24, 25, 27) in rotation, the rotor (23) comprising external teeth (26) which can mesh with an external gearing corresponding toothing of the driving shaft (41).

2. Removable cartridge (10) according to claim 1, **characterised in that** the bore (80) comprises at least a first groove (83) and a second groove (84), the first groove (83) being used to accommodate a first damping seal (12) and the second groove (84) being used to accommodate a second damping seal (12).

3. Dental and/or surgical instrument (1) comprising a motor driving a tool via a tool holder (24, 25, 27) located in the head of the dental and/or surgical instrument (1), and a removable cartridge (10) according to claim 1, such that the tool holder (24, 25, 27) is supported in the head of the dental and/or surgical instrument (1) by the first ball bearing (21) and the second ball bearing (22) of the removable cartridge (10).

4. Dental and/or surgical instrument (1) according to claim 3, **characterised in that** the cartridge (10) is locked by a nut (18).

5. Dental and/or surgical instrument (1) according to claim 3 or 4, **characterised in that** the cartridge (10) is positioned and locked in the head of the dental and/or surgical instrument (1) by means of the end of the driving shaft (41).

6. Dental and/or surgical instrument (1) according to one of claims 3 to 5, **characterised in that** the central axis of the driving shaft (41) is perpendicular to the shaft of the rotor (23).

7. Dental and/or surgical instrument (1) according to one of claims 3 to 5, **characterized in that** the outer casing (30) of the dental and/or surgical instrument (1) is made of a material different from the material of the cartridge (10).
